# EUROPEAN PATENT APPLICATION

(11) **EP 0 604 744 A2**
(43) Date of publication of application: **06.07.1994**
(21) Application number: 93118259.6
(22) Date of filing: 11.11.1993
(51) Int. Cl.: A61K 31/00, A61K 31/195, A61K 31/425, A61K 37/02

(54) **Method for treating systemic inflammatory response syndrome**

(30) Priority: 30.11.1992 US 983415
(71) Applicant: Transcend Therapeutics, Inc., Cambridge Massachusetts 02139 (US)
(72) Inventor: Goldberg, Dennis I., Hawthorn Woods, IL 60047 (US); Mark, David A., Oak Park, IL 60304 (US); Pace, Gary W., Northfield, IL 60093 (US)
(74) Representative: MacGregor, Gordon

(57) **Abstract**

The present invention provides a therapy and composition for the treatment of systemic inflammatory response syndrome and the prevention of same. Pursuant to the present invention, a composition is administered for stimulating the intracellular synthesis of glutathione. At the same time, the patient's arginine intake is limited. Preferably, the arginine intake is limited to less than 0.4% of the patient's total calories.

## Description

### BACKGROUND OF THE INVENTION

The present invention relates generally to compositions for the treatment of disease states. More specifically, the present invention relates to compositions for the treatment of systemic inflammatory response syndrome and/or the prevention of same.

Sepsis has been described as the systemic response to infection. It is an increasingly common cause of morbidity and mortality and has been reported to be the most common cause of death in non-coronary intensive care units. See, Definition for Sepsis and Organ Failure, *Critical Care Medicine*, Vol. 20, No. 6, p. 864, *et. seq.* (1992).

Systemic inflammatory response syndrome describes wide-spread inflammation that occurs in patients with such diverse disorders as infection, pancreatitis, ischemia, multi-trauma, hemorrhage shock, or immunologically mediated organ injury. Sepsis is a sub-category of this dysfunction. See, "Definition for Sepsis and Organ Failure," *Critical Care Medicine*, Vol. 20, No. 6, pp. 724-725.

When systemic inflammatory response syndrome is due to infection, the terms "sepsis" and "systemic inflammatory response syndrome" are synonymous. A frequent complication of systemic inflammatory response syndrome is the development of organ system dysfunction. These include conditions, such as: acute lung injury; shock; renal failure; and multiple organ dysfunction syndrome. Supra.

Severe sepsis, which can lead to septic shock, is sepsis associated with organ dysfunction, hypoperfusion, or hypotension. Septic shock is defined as sepsis with hypotension, along with the presence of perfusion abnormalities. Supra.

Large scale multi-center, sepsis studies have indicated that there is a continuum of severity that has both infectious and inflammatory components. The condition begins with infection and can lead to sepsis with organ dysfunction and septic shock. Supra, citing: Bone RC, Risher CJ Jr., Clemmer TP, et al: A Controlled Clinical Trial of High-Dose Methylprednisolone in the Treatment of Severe Sepsis and Septic Shock, *N Engl J Med* 1987; 317:653-658; The Veterans Administration Systemic Sepsis Cooperative Study Group, Effect of High-Dose Glucocorticoid Therapy on Mortality in Patients with Clinical Signs of Systemic Sepsis, *N Engl J Med* 1987; 317:659-665; Ziegler EJ, Fisher CJ Jr, Sprung CL, et al: Treatment of Gram-Negative Bacteremia and Septic Shock With HA-1A Human Monoclonal Antibody Against Endotoxin, *N Engl J Med* 1991; 324:429-436; and Greenman RL, Schein RMH, Martin MA, et al: A Controlled Clinical Trial of E5 Murine Monoclonal 1 gM antibody to Endotoxin in the Treatment of Gram-Negative Sepsis, *JAMA* 1991; 266:1097-1102.

Septic shock is associated with increased mortality rate. Supra, citing: Bone RC, Fisher CJ Jr, Clemmer TP, et al: Sepsis Syndrome: A Valid Clinical Entity, *Crit Care Med* 1989; 17:389-393; Kreger BE, Craven DE, McCabe WR: Gram-Negative Bacteremia IV. Re-Evaluation of Clinical Features and Treatment in 612 Patients, *Am J Med* 1980; 68:344-350; and Weil MH, Shubin H, Biddle M: Shock Caused by Gram-Negative Microorganisms, *Ann Intern Med* 1980; 60:384-400. Despite the use of innovative therapies, the morbidity and mortality rates in severe sepsis remains high.

### SUMMARY OF THE INVENTION

The present invention provides a therapy and composition for the treatment of systemic inflammatory response syndrome and the prevention of same. Pursuant to the present invention, a composition is administered for stimulating the intracellular synthesis of glutathione. At the same time, the patient's arginine intake is limited. Preferably, the arginine intake is limited to less than 0.4% of the patient's total calories.

It has been found that systemic inflammatory response syndrome includes among its symptoms oxidative damage from free radicals and profound hypotension. By reducing the arginine intake in the patient while administering a composition for stimulating the intracellular synthesis of glutathione, synergistic advantages are achieved.

Accordingly, the present invention provides for the use of an intracellular glutathione stimulator for creating a composition that is provided to a patient for treating sepsis or systemic inflammatory response, while at the same time the arginine intake of the patient is reduced.

Preferably, the intracellular glutathione stimulator is chosen from the group consisting of: L-2-oxothiazolidine-4-carboxylate; esters of 2-oxothiazolidine-4-carboxylate; glutathione esters; N-acetylcysteine; and cysteine-rich proteins.

The present invention can also be used prophylactically for those patients in danger of systemic inflammatory response syndrome.

Pursuant to the present invention, in an embodiment, a complete nutrition formulation is provided that has reduced, or no arginine and includes an intracellular glutathione stimulator. On the other hand, in an embodiment of the present invention, the intracellular glutathione stimulator can be provided separately and the patient can be provided nutritional requirements through other means, but, the patient's arginine intake is restricted.

An advantage of the present invention is that it provides a therapy for treating systemic inflammatory response or other septic conditions.

A further advantage of the present invention is that it provides a composition wherein less nitric oxide is available for the formation of nitrosocysteine, nitrosoglutathione, or other nitrosothiols.

Still further, an advantage of the present invention is that less nitric oxide is available to be directly converted into free radicals, such as ONOO-.

Furthermore, an advantage of the present invention is that a reduction in nitric oxide lessens the signals (such as cyclic GMP) for continuing release of cytokines.

Additionally, an advantage of the present invention is that less arginine is available for incorporation into defensin polypeptides by neutrophils.

Further, an advantage of the present invention is that it provides a therapy for preventing systemic inflammatory response syndrome in a patient at risk of same.

Moreover, an advantage of the present invention is that it allows intracellular glutathione levels to be increased without the risk of increasing vasodilation and other inflammatory responses.

Additional features and advantages of the present invention are described in, and will be apparent from, the detailed description of the presently preferred embodiments and from the drawings.

### BRIEF DESCRIPTION OF THE DRAWINGS

Figure 1 illustrates schematically the flow of arginine within the body and into a cell and pathways involved.

Figure 2 illustrates a pathway of the conversion of arginine in a cell.

Figure 3 illustrates schematically the interaction of L-2-oxothiazolidine-4-carboxylate and arginine in a cell and some of the pathways involved.

### DETAILED DESCRIPTION OF THE PRESENTLY PREFERRED EMBODIMENTS

The present invention provides a therapy for treating systemic inflammatory response syndrome and sepsis. Additionally, the present invention provides a composition for preventing systemic inflammatory response syndrome in a patient at risk of same.

Pursuant to the present invention, the patient is administered a therapeutically effective amount of an intracellular glutathione stimulator while, at the same time, the intake of arginine by the patient is limited. The non-essential amino acid arginine is converted to citrulline and nitric oxide by the enzyme nitric oxide synthetase (NOS) in the body. Typically, the availability of arginine is not limiting to the reaction. However, this may be the case when nitric oxide synthetase is induced by cytokines or other signals.

Acute systemic infection or inflammation, including sepsis, results in an increase in the production of cytokines by white blood cells, including tumor necrosis factor. Cytokines, and especially tumor necrosis factor, increase the production of nitric oxide. An abundance of nitric oxide can cause vasodilation leading to systemic hypotension. In a septic patient, the result can be severe sepsis due to sepsis-induced hypotension.

Nitric oxide production can also be a concern with respect to certain other inflammatory reactions, such as Crohn's disease and colitis. Such chronic inflammatory disorders can produce cytokines resulting in an increase in nitric oxide. The nitric oxide adds to the inflammatory reaction.

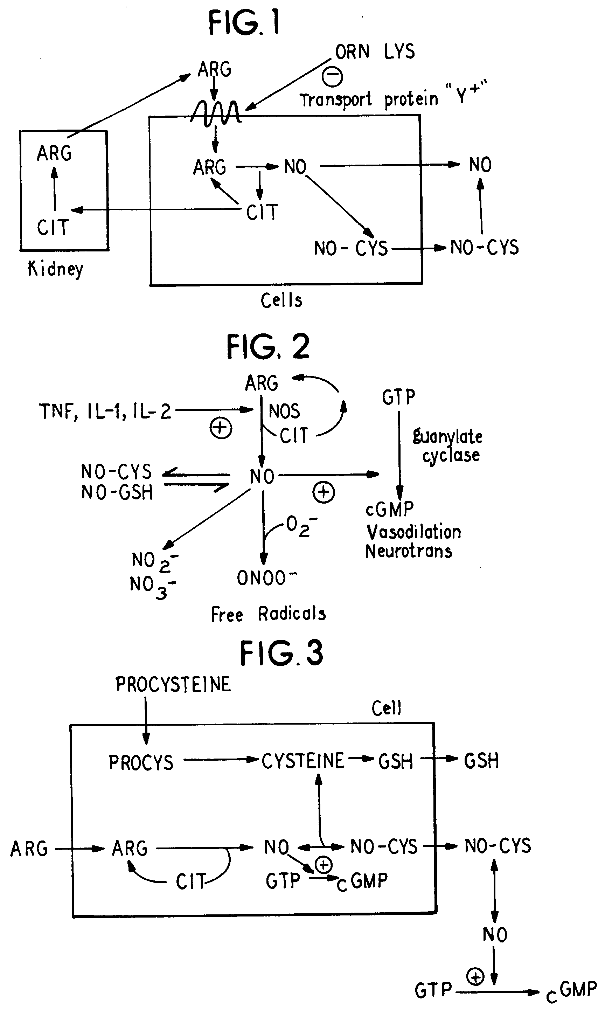

Referring to Figure 1, the transport of arginine into a cell is illustrated as well as some of the intracellular pathways. As illustrated, arginine is transported into the cell via transport protein "Y+." Ornithine and lysine compete with arginine for transport protein "Y+."

As illustrated in detail in Figure 2, once in the cell, a pathway of arginine results in the generation of nitric oxide. The nitric oxide can then generate cGMP a vasodilation neurotransmitter. Additionally, free radicals ONOO- are generated, as well as NO₂⁻ and NO₃⁻. The nitric oxide will also interact with cysteine and glutathione to generated nitrosocysteine (NO-CYS) and nitrosoglutathione (NO-GSH).

Although, due to the generation of free radicals and the risk of oxidative damage, it may be desirable to increase the intracellular synthesis of glutathione, this can worsen the condition. Figure 3 illustrates the potential intracellular mechanism of L-2-oxothiazolidine-4-carboxylate, a glutathione precursor, when it is added to the cell.

L-2-oxothiazolidine-4-carboxylate is converted intracellulary to cysteine and then glutathione. However, the cysteine will react with the nitric oxide generated by the arginine pathway resulting in NO-CYS and cGMF. This will increase vasodilation and the inflammatory response. The generation of NO-CYS, nitrosoglutathione, and nitrosothiols are seen as having or contributing to the effects attributed to nitric oxide. The effects include, hypotension, increased cytokine production, and neurological disorders.

The inventors have found that by limiting the patient's intake of arginine, the conversion of arginine to nitric oxide is sufficiently reduced that an intracellular glutathione stimulator can also be administered without increasing the risk of hypotension and other inflammatory responses. The arginine intake is preferably limited to less than 0.4% of the total calories. Most preferably, the arginine content is limited to less than 0.2%. In an embodiment, the patient does not receive any arginine.

In other respects, the patient's dietary intake can be nutritionally complete. If desired, citrulline and ornithine can be administered to the patient. These arginine-derived substances may help to preserve the urea cycle without contributing to nitric oxide formation. Further, ornithine competes with arginine for transport protein "Y+" further limiting nitric oxide generation. Likewise, lysine can be administered to limit nitric oxide generation.

As previously stated, pursuant to the present invention, an intracellular glutathione stimulator is administered to the patient. There are a number of potential intracellular glutathione stimulators.

In a preferred embodiment, L-2-oxothiazolidine-4-carboxylate is administered to the patient. L-2-oxothiazolidine-4-carboxylate is subjected to the action of 5-oxo-L-prolinase in the presence of adenosine triphosphate to produce S-carboxyl cysteine. S-carboxyl cysteine is then decarboxylated to produce cysteine. Cysteine is then metabolized to produce glutathione. See, U.S. Patent Nos.: 4,335,210; 4,434,158; 4,438,124; 4,665,082; and 4,647,571, the disclosures of which are incorporated herein by reference.

The inventors also believe that other substances that stimulate intracellular glutathione synthesis can be utilized. To this end, esters of 2-oxothiazolidine-4-carboxylate can also be used. The ester is a saturated straight or branched, alkyl group of 1 to 10 carbon atoms. Preferably, the ester is chosen from a saturated straight alkyl group such as methyl, ethyl, propyl, butyl, pentyl, hexyl, heptyl, octyl, nonyl, or decyl and a saturated branched alkyl group such as isopropyl, isobutyl, sec-butyl, tert-butyl, or isopenyl. Esters of 2-oxothiazolidine-4-carboxylate are disclosed in U.S. patent application Serial No. 07/932,761, filed on August 20, 1992, the disclosure of which is hereby incorporated by reference.

Additionally, in an embodiment, a glutathione ester can be used to stimulate intracellular glutathione synthesis. Such a glutathione ester, for example, can have the structure: 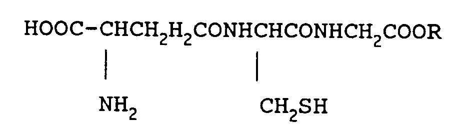
wherein R is an alkyl group containing 1 to 10 carbon atoms. Preferably, the methyl and ethyl glutathione esters are used. It is also preferred to use glutathione isopropyl ester. Glutathione esters are disclosed in U.S. Patent No. 4,784,685, the disclosure of which is incorporated herein by reference.

Further, N-acetylcysteine can be utilized to stimulate the intracellular synthesis of glutathione.

The intracellular glutathione stimulator can also comprise a protein. The protein must have a sufficiently high cysteine content to stimulate the intracellular synthesis of glutathione. Preferably, the protein is a "cysteine-rich protein." As used herein, a "cysteine-rich protein" means a protein that is at least approximately 1.3% by weight cysteine. This excludes the protein from being merely, casein, total milk product, or soy protein. On the other hand, whey (2.0%), egg white (2.5%), serum albumin (5.5%), and lactalbumin (5.8%) all have sufficient cysteine content, as well as mixtures including same.

Pursuant to the present invention, a therapeutically effective amount of an intracellular glutathione stimulator is administered to a patient having systemic inflammatory response syndrome or at risk of same. At the same time, the patient's arginine intake is limited.

If desired, a complete nutritional formulation, either enteral or parenteral, that incorporates the intracellular glutathione stimulator can be provided.

By way of example, and not limitation, an example of a complete enteral formulation including the intracellular glutathione stimulator is as follows: a liquid, ready-to-use enteral product with protein at 20% of total calories: half from a cysteine rich protein source such as partially hydrolyzed lactalbumin and half from free amino acids, optionally including ornithine and/or citrulline. Carbohydrates would be 40-50% of calories. Lipids comprise 30-40% of calories, preferably a blend of medium chain triglycerides, marine oils, vegetable oils and lecithin. Vitamin and mineral content would meet preferably daily requirements in 1000 calories. This product would contain less than 0.2% of calories as arginine and more than 0.4% of calories as cysteine (both from the lactalbumin protein).

An example of a complete parenteral formulation including the intracellular glutathione stimulator is as follows: protein is provided in the form of free amino acids as follows: isoleucine 9%, leucine 14%, valine 8%, lysine 9%, methionine 4%, phenylalanine 5%, threonine 5%, tryptophan 1%, alanine 6%, aspartate 9%, glutamate 11% glycine 4%, histidine 2%, proline 8%, serine 4%, tyrosine 1%, and arginine 0%. L-2-oxothiazolidine-4-carboxylate at 4-10 grams/day is either mixed with the amino acid solution for simultaneous administration or administered separately.

On the other hand, the therapy can merely include administering a therapeutically effective amount of the intracellular glutathione stimulator while limiting the patient's intake of arginine. Examples of enteral diets for limiting arginine intake are disclosed in U.S. patent application entitled: "COMPOSITION AND METHOD FOR REDUCING THE RISK OF HYPOTENSION", assigned to the assignee of the instant patent application and filed on November 5, 1992. The disclosure of which is incorporated herein by reference.

Likewise, a complete parenteral formulation having a reduced arginine content can be administered with the intracellular glutathione stimulator being administered separately. For example, a 3% L-2-oxothiazolidine-4-carboxylate product in a buffer solution can be used parenterally.

By way of example, and not limitation, contemplative examples of the use of the present invention are as follows:

### EXAMPLE NO. 1

Two hundred patients admitted to intensive care units and considered at high risk to develop systemic inflammatory response syndrome (SIRS) are nutritionally supported by the use of a tube-fed enteral formula. Half receive a whole protein based formula with typical arginine and cysteine concentrations. Half receive an arginine-poor, cysteine-rich formula containing hydrolyzed lactalbumin and free amino acids.

Thirty percent in each group develop classically defined SIRS, but the severity of the hypotension and the duration of the condition are less in the latter group. One benefit of providing nutrients other than arginine is that catabolism of muscle and other tissues is reduced, in turn reducing the arginine released from these tissue pools. Nitric oxide production is curtailed by the restriction on available arginine.

### EXAMPLE NO. 2

Fifty patients with SIRS are switched from a typical amino acid solution containing 8-10% of protein as arginine to a formulation without any arginine. Optionally the new solution contains about 2% of protein as ornithine. The patients also receive a separate parenteral infusion of a 3% L-2-oxothiazolidine-4-carboxylate product at 3-12 grams every 24 hours. The survival rate in this group is better than typically expected for patients with SIRS diagnosis.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. The use of a composition having a therapeutically effective amount of an intracellular glutathione stimulator for the preparation of a composition that is administered to a patient having sepsis or systemic inflammatory response syndrome while at the same time the arginine intake of the patient is reduced.

2. The use of a composition having a therapeutically effective amount of an intracellular glutathione stimulator for the preparation of a composition that is administered to a patient for treating oxidative damage and limiting the generation of nitroso-substances in a patient at risk of systemic inflammatory syndrome, the composition being administered while the arginine intake of the patient is limited to 0.4% or less of the total calorie intake of the patient.

3. The use according to Claims 1 or 2 wherein the intracellular glutathione stimulator is chosen from the group consisting of: L-2-oxothiazolidine-4-carboxylate; esters of 2-oxothiazolidine-4-carboxylate; glutathione esters; N-acetylcysteine; and cysteine rich proteins.

4. The use according to Claim 1 wherein the patient's arginine intake is limited to approximately 0.4% or less arginine as a percent of the total calories.

5. The use according to Claims 1 or 2 comprising the further step of administering at least one of ornithine, citrulline, or lysine to the patient.

6. The use according to Claims 1 or 2 wherein the intracellular stimulator is administered as part of a complete nutritional composition to the patient.

7. The use according to Claims 1 or 2 wherein the intracellular glutathione stimulator is administered enterally.

8. The use according to Claims 1 or 2 wherein the intracellular glutathione stimulator is administered parenterally.

9. The use according to Claims 1 or 2 wherein the patient receives no arginine.

10. An enteral composition comprising:
a protein source;
a carbohydrate source;
a fat source;
an intracellular glutathione stimulator chosen from the group consisting of L-2-oxothiazolidine-4-carboxylate, esters of 2-oxothiazolidine-4-carboxylate, glutathione esters, and N-acetylcysteine; and
wherein less than or equal to 0.2% of the calories are from arginine.

11. The enteral composition of Claim 10 wherein the composition includes no arginine.

12. A parenteral composition comprising:
a protein source;
a carbohydrate source;
a fat source;
an intracellular glutathione stimulator chosen from the group consisting of L-2-oxothiazolidine-4-carboxylate, esters of 2-oxothiazolidine-4-carboxylate, glutathione esters, and N-acetylcysteine; and
wherein less than or equal to 0.2% of the calories are from arginine.

13. The parenteral composition of Claim 12 wherein the composition includes no arginine.
